# EUROPEAN PATENT SPECIFICATION

(11) **EP 2 037 958 B1**
(45) Date of publication and mention of the grant of the patent: **01.02.2012**
(21) Application number: 07766931.5
(22) Date of filing: 15.06.2007
(51) Int. Cl.: A61K 39/008, C12N 5/16, A61P 33/02

(54) **A HYBRID CELL VACCINE AGAINST LEISHMANIASIS [KALA-AZAR]**
HYBRID-ZELL-VAKZINE GEGEN LEISHMANIASIS KALA-AZAR
VACCIN A CELLULES HYBRIDES CONTRE LA LEISHMANIOSE [KALA-AZAR]

(30) Priority: 15.06.2006 IN DE14292006
(43) Date of publication of application: 25.03.2009
(73) Proprietor: Council of Scientific and Industrial Research, New Delhi - 110 001 (IN)
(72) Inventor: BHAUMIK, Suniti, Kolkata 700 032, West Bengal (IN); BASU, Rajatava, Kolkata 700 032, West Bengal (IN); NASKAR, Kshudiram, Kolkata 700 032, West Bengal (IN); ROY, Syamal, Kolkata 700 032, West Bengal (IN)
(74) Representative: Webber, Philip Michael
(86) International application number: PCT/IN2007/000243
(87) International publication number: WO 2007/144903

(56) References cited:
- EP-A- 1 130 088
- BASU RAJATAVA ET AL: "Kinetoplastid membrane protein-11 DNA vaccination induces complete protection against both pentavalent antimonial-sensitive and resistant strains of Leishmania donovani that correlates with inducible nitric oxide synthase activity and IL-4 generation: Evidence for mixed Th1-and Th2-like responses i" JOURNAL OF IMMUNOLOGY, vol. 174, no. 11, June 2005 (2005-06), pages 7160-7171, XP002470914 ISSN: 0022-1767
- BASU RAIATAVA ET AL: "Hybrid cell vaccination resolves Leishmania donovani infection by eliciting a strong CD8(+) cytotoxic T-lymphocyte response with concomitant suppression of interleukin-10 (IL-10) but not IL-4 or IL-13" INFECTION AND IMMUNITY, vol. 75, no. 12, December 2007 (2007-12), pages 5956-5966, XP008089010 ISSN: 0019-9567

## Description

The present invention relates to a hybrid cell vaccine against Leishmaniasis comprising a syngeneic macrophage with a dominant *Leishmania* antigen-KMP-11 electrofused with allogenic dendritic cells, particularly for use against visceral leishmaniasis. The vaccine of the present invention was able to achieve complete clearance of splenic and hepatic parasite burden in late stage of *L. donovani* infection in genetically susceptible BALB/c mice.

Visceral leishmaniasis (VL), also known as Kala-azar, is caused by members of *L*. *donovani* complex resulting in clinical symptoms like fever, cachexia, hepatosplenomegaly and blood cytopenia. Active VL is associated with the absence of parasite specific cell mediated immune response (9, 10). VL has also been increasingly recognized as an opportunistic infection in individuals infected with HIV virus (11). WHO has identified leishmaniasis as a major and increasing public health problem (12). Visceral form of leishmaniasis is fatal if left untreated with recent epidemics in Sudan and India resulting in more than 100,000 deaths (13).

Present forms of chemotherapeutic regimen against Kala-azar worldwide have severe limitations due to toxic effect, prolonged duration of treatment, and in some cases prohibitive high cost of treatment Failure of the pentavalent antimonials- the present main form of chemotherapeutic treatment worldwide, is attributed to the emergence of antimony resistant *Leishmania* strains resulting in frequent relapses after treatment (14, 15). In India, antimony is no longer useful as a drug as 65% of VL patients fail to respond or promptly relapse (16). Alternative chemotherapeutic treatments with amphotericin B and its lipid formulation have severe limitations due to toxic effect and prohibitive high cost of treatment (15).

Growing limitations in available chemotherapeutic strategies due to emerging resistant strains and lack of an effective therapeutic vaccine strategy against VL deepens the crisis. Therefore, attempts were made to use hybrid cell therapy approach against visceral Leishmaniasis. Genetically engineered Dendritic Cells [DC] based vaccines represent a powerful tool for immunotherapy, although so far it has been implemented in cancer model only. The present invention for the first time substantiated that DC-based hybrid cell therapy can be implemented in future vaccine designs against advanced state of Visceral Leishmaniasis [VL] and has the potential to be used in intracellular parasitic disease in general.

A realistic assessment of efficacy of vaccine against Leishmaniasis depends upon 3 important variables that we considered in the present invention - (i) genetic make up of the host; (ii) nature of the antigen tested; (iii) nature of vaccine. Considering the first variable, the goal of this invention was to formulate a vaccine strategy that was based on its implementation in golden hamster model as they largely reflect clinico-pathological features of progressive human VL, showing a relentless increase in visceral burden, progressive cachexia, hepatosplenomegaly, pancytopenia, hypergammaglo-bulinemia and ultimately death (1).

Address to the second variable was based on selection of Kinetoplastid Membrane Protein-11 [KMP-11] as a vaccine candidate antigen. Kinetoplastid Membrane Protein-11 (KMP-11), a highly conserved surface membrane protein present in all members of the family Kinetoplastidae, is differentially expressed both in amastigote and promastigote forms of *Leishmania* (2 - 4). In a previous report, implication towards an association of KMP-11 expression with the vaccine potential of an attenuated, avirulent *Leishmania* strain-UR6 (5) has been made. Moreover, the ability of KMP-11 to induce IFN-γ from PBMC derived from cured Kenyan VL patients has been reported (6). Several findings indicate towards a disparate host response to different parasite antigens in cutaneous and visceral forms of leishmaniasis. *Leishmania* glycoprotein-63 (gp63) failed to induce significant IFN-γ from lymphocytes of patients cured from VL (6). On the other hand, the same antigen, gp63, induced high levels of IFN-γ from lymphocytes of cured CL patients (8). Unlike gp63, KMP-11 induced significant production of IFN-γ from lymphocytes of cured VL patients (6). Thus, the nature of antigen influencing the host immune response to different *Leishmania* species comes under scrutiny.

Keeping in view, the hitherto known facts that the transfected plasmid encoding the immunodominant antigen is expressed through class I pathway and that DCs are most important members of general class of APC, the inventors of the present invention realized that the transfection of plasmid encoding KMP-11 antigen might help focus the immune response to generate antigen specific CTLs against this defined immunodominant antigen. Furthermore, the antigenicity of transfected APC is expected to be boosted by contribution of allogeneic MHC class II to bring about more potent Tₕ response and effector mechanism in terms of CTL generation mediated by a common cell displaying epitope for syngeneic MHC class I and allogeneic class II molecules. Therefore, in the present invention a genetically engineered DC based hybrid cell therapy in experimental VL model, obtained by transfecting DC with the dominant KMP-11 antigen is provided.

EP 1130088 relates to methods for treating and preventing cancer and infectious disease using hybrid cells formed by fusion of allogeneic dendritic cells and autologous non-dendritic cells. Such hybrid cells are said to combine the vigorous alloreactivity of mature DCs with the specific antigenicity of autologous tumour cells, thereby eliciting a highly specific and vigorous CTL response. The methods disclosed therein are said to be capable of treating or preventing infections caused by pathogenic protozoans such as, but not limited to, *Entomoeba histolytica, Trichomonas tenas, Trichomonas hominis, Trichomonas vaginalis, Trypanosoma gambiense, Trypanosoma rhodesiense, Trypanosoma cruzi, Leishmania donovani, Leishmania tropica, Leishmania braziliensis, Pneumocystis pneumonia, Plasmodium vivax, Plasmodium falciparum,* and *Plasmodium malaria.*

Thus, the main object of the present invention is to provide a hybrid cell vaccine against Leishmaniasis comprising syngeneic macrophage with a dominant *Leishmania* antigen-KMP-11 electrofused with allogenic dendritic cells.

Another object of the present invention is to provide a hybrid cell vaccine wherein the *Leishmania* antigen-KMP-11 is obtained from *Leishmania donovani.*
Another object of the present invention is to provide a vaccine wherein the vaccine stimulates antibody response, a CTL response, T -cell proliferative response, IL-2 generation, reduction of splenic and liver parasite burden along with sterile cure against the *Leishmania* upon therapeutic vaccination to a mammal.

Another object of the present invention is to provide a vaccine wherein the said vaccine is capable of protecting the spleen and-liver from parasites up to six times of the effective dose of the said vaccine.

Yet another object of the present invention is to provide a hybrid vaccine formulation against Leishmainiasis comprising a syngeneic macrophage with a dominant *Leishmania* antigen-KMP-11 electrofused with allogenic dendritic cells along with an adjuvant and/or cytokine.

A further object of the present invention is to provide a process for the preparation of a hybrid cell vaccine against leishmaniasis.

A hybrid cell vaccination approach with dendritic cells has been widely used successfully in tumor models only. By devising a novel strategy of transfecting syngeneic macrophage cell lines from BALB/c mice with a dominant *Leishmania* antigen-KMP-11 and electrofusing with allogenic DC from C57BU6, the inventors obtained a complete clearance of splenic and hepatic parasite burden in late stage *L*. *donovani* infection in genetically susceptible BALB/c mice. Curative response resulted from a high KMP-11 specific MHC class I restricted CD8⁺CTL response. This is the first study that realized the potential of hybrid cell vaccine in the field of infectious disease and clearly shows the contribution of Th2 cytokine response in conferring protection to *Leishmania donovani* infection in experimental VL model.

Accordingly, the present invention provides a hybrid cell vaccine against Leishmaniasis comprising of syngeneic macrophage with a dominant *Leishmania* antigen-KMP-11 electrofused with allogenic Dendritic Cell.

The invention further provides a hybrid vaccine formulation against Leishmainiasis comprising a syngeneic macrophage with a dominant *Leishmania* antigen-KMP-11 electrofused with allogenic dendritic cells along with an adjuvant and/or cytokine.

The invention also provides a process for the preparation of a hybrid cell vaccine against leishmaniasis as claimed in claim 9.
In an embodiment the present invention provides a hybrid cell vaccine comprising fusion of syngeneic BMDM expressing parasite surface antigen known as Kinetoplastid Membrane Protein-11 (KMP-11) with allogeneic bone marrow derived dendritic cells (BMDC).

In another embodiment of the present invention, the macrophage is obtained from BALB/c mice by known methods.

In yet another embodiment of the present invention, the *Leishmania* antigen-KMP-11 gene is obtained from *Leishmania donovani.*

In still another embodiment of the present invention, the allogenic dendritic cell is obtained from bone marrow progenitors by known methods.

In a further embodiment of the present invention, the vaccine elicits a cellular immune response which is a mixed Th1/Th2 cell response

In another embodiment of the present invention, the vaccine stimulates KMP-11 specific CD8+ cytotoxic T cell response.

In yet another embodiment of the present invention, the vaccine stimulates an antibody response, a CTL response, T-cell proliferative response, IL-2 generation, reduction of splenic and liver parasite burden along with sterile cure against *Leishmania* upon therapeutic vaccination to a mammal.

In still another embodiment of the present invention, the administration of the vaccine is effective to eliminate the parasite from the mammal.

In another embodiment, the present invention provides a hybrid vaccine formulation against leishmaniasis comprising of syngeneic macrophage with a dominant *Leishmania* antigen-KMP-11 electrofused with allogenic dendritic cell optionally along with an adjuvant and/or cytokine.

In a further embodiment, the present invention provides a pharmaceutical formulation comprising the aforesaid hybrid cell vaccine optionally along with a carrier or excipient.

In yet another embodiment of the present invention, the allogeneic antigen presenting cells (APC), are bone marrow derived dendritic cells (BMDC) obtained from C57BU6 mouse strain.

In still another embodiment of the present invention, the antigen comprises an immunodominant cell surface protein of *Leishmania* KMP-11.

In an embodiment of the present invention, the therapeutic administration of the hybrid cell vaccine is effective to eliminate parasites from organs of the mammals infected with *Leishmania.*

In another embodiment of the present invention, the strain of *Leishmania* is *L. donovani.*

Also disclosed is a cDNA which is a polynucleotide comprising a coding region encoding the antigen and the antigen is a cell surface membrane protein of *Leishmania.*

Also disclosed is a method for the treatment of leishmaniasis, comprising administering to the subject in need thereof an effective, non-toxic amount of the vaccine formulation as claimed in claim 7.

A further embodiment of the present invention provides a hybrid cell vaccine of the invention, the therapeutic administration of which is effective to eliminate parasites from organs of the mammals infected with *Leishmania.*

In another embodiment of the present invention, the therapeutically effective intra venous dose of the said vaccine is 5 x 10⁵ cells/100g body wt./100µl PBS with 5 days interval.

In yet another embodiment of the present invention, the said dose is inoculated as a series of doses over a period of nearly four weeks.

Another embodiment of the present invention provides a hybrid cell vaccine of the invention, the therapeutic administration of which is effective for obtaining complete clearance of splenic and hepatic parasite burden in late stage infection from the parasite.
KMP-11, *Kinetoplastid Membrane Protein-11;*
LD, *Leishmania donovani;*
VL, *Visceral Leishmaniasis;*
CL, *Cutaneous Leishmaniasis;*
SLA *Soluble Leishmanial Antigen,*
BMDM, bone marrow derived macrophage;
BMDC bone marrow derived dendritic cells
APC allogeneic antigen presenting cells

### BRIEF DESCRIPTION OF DRAWINGS AND FIGURES:

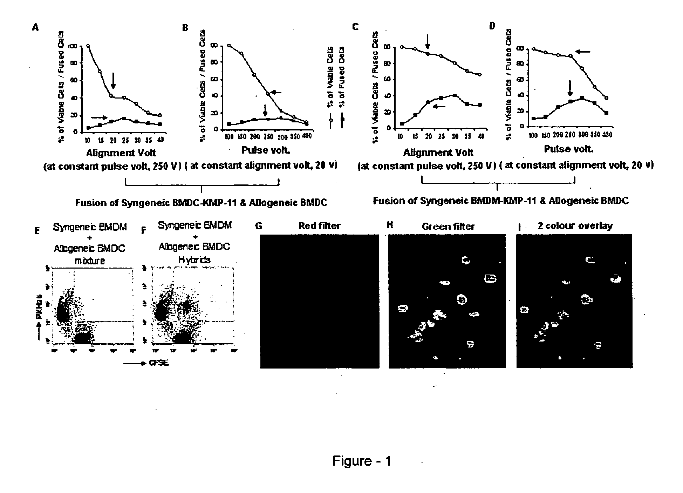

**Figure 1** **A, B, C & D:** Standardization and optimization of Electro fusion Parameters for hybrid cells generation.
**Figure 1** **E & F:** Calculation of the generation of hybrids: Cells were analysed by flow cytometry and % of viable hybrid cell generation was calculated from double positive cells in the third quadrant of a dot plot.
**Figure 1G****, H & I:** Fluorescence micrographs of hybrid cells generated by electrofusion-
   (G) Field seen through red filter
   (H) field seen through green filter and
   (I) finally two colour overlay was performed to enable visualization of double-positive cells.
**Figure 2****:** Assay of Organ parasite burden by limiting dilution assay method. Total organ parasite burden was calculated from spleen or liver weight. Data represent the mean ± SE for 12 animals per group.
**Figure 3****:** Anti-KMP-11 CTL response in hybrid cells or KMP-11 DNA transfected syngeneic BMDC vaccinated 105 days *L*. *donovani* post infected mice compared to respective control groups
**Fig 4****:** T cell proliferation, IL-2 and IL-4 assay in response to SLA stimulation between different groups of experimental mice.
**Figure 5** **A, 5 B & 5 C:** Anti-KMP-11 whole IgG, IgG1 and IgG2 Ab titers in the sera of hybrid cells or KMP-11 DNA transfected syngeneic BMDC treated 105 days AG83 infected BALB/c mice (n=5/group).
   **Figure 5D****:** Ratio of anti-KMP-11 IgG2a/IgG1 titers in the sera from different infected and therapeutically vaccinated BALB/c mice.

### DETAILED DESCRIPTION OF THE INVENTION

### MAKING OF HYBRID CELL

### 1. Cloning of KMP-11

10⁸ promastigotes washed with PBS, pH 7.4, (1.37 mM NaCl, 2.7 mM KCI, 4.3 mM Na₂HPO₄, 1.47 mM KH₂PO₄) and STE (10 mM Tris-HCl, pH 7.5, 100 mM NaCl, 1 mM EDTA) were suspended in STE buffer and incubated with Proteinase K (1 mg/ml) (Invitrogen, Grand Island, NY), 0.5% SDS at 50°C for 4 h. Nucleic acids were extracted by phenol: chloroform: isoamyl alcohol extraction and ethanol precipitation. Genomic DNA was spooled, and subjected to RNase (100 µg/ml) treatment. Genomic DNA from muscles was extracted as described previously (16). Whole DNA from all tissues was extracted with Extraction Buffer (10 mM Tris pH 8.0, 0.1 M EDTA, 20 □g/ml pancreatic RNase, 0.5% SDS and 100 □g/ml Proteinase K) followed by phenol: chloroform: isoamyl alcohol extraction and ethanol precipitation method. All the PCR amplification of KMP-11 DNA was done using KMP-11 gene-specific primers: forward primer 5'-ATGGCCACCACGTACGAGGAG-3' and reverse - primer 5'-TTACTTGGACGGGTACTGGGC-3' with initial denaturation at 94°C for 5 min followed by 35 cycles of denaturation at 94°C for 30 s, primer annealing at 60°C for 30 s, and extension at 72°C for 30 s. pCMV-LIC vector used for cloning was purchased from Pharmingen (San Diego, CA).

KMP-11 gene from *L*. *donovani* genomic DNA was amplified using High Fidelity Taq^{™} Polymerase (Invitrogen, Grand Island, NY) having a 3'-5' exonuclease activity. PCR reactions were performed using the following reagents: 50 mM Tris-Cl, 1:5 mM MgCl₂ and 10 µM of each dNTPs ( Invitrogen, Grand Island, NY) and KMP-11 specific primers with an additional 13 bp sequence homology to the vector plasmid, forward: 5' CTGGTTCCGGCGAATGGCCACCACGTACGAGGAG 3'; reverse: 5' CTCGCTCCGGCGATTACTTGGACGGGTACTGCGC 3' for 35 cycles in a Thermocycler (Gene Amp^{®} PCR System 9700, AB Applied Biosciences) under conditions: at 94°C for 2 min, 94°C for 30 s, 62°C for 30 s, and 72°C for 30 s. Amplified PCR products were electrophoresed in agarose gel and eluted from the gel by GenElute^{™} Minus EtBr Spin Columns (Sigma, St. Louis, MO). For the generation of vector compatible 13 bp single stranded overhangs, 100 µg purified DNA was treated with 5U T4 DNA polymerase (Invitrogen, Grand Island, NY) in a 10 µl reaction volume (165 mM Tris acetate, 330 mM sodium acetate, 50 mM magnesium acetate, 2.5 mM DTT, and 100µg/ml BSA) in the presence of 0.5 mM dTTP at 37°C for 20 min. The linear pCMV-LIC (7.5 ng) with 13 bp single-stranded homologous sequence and T4 DNA Polymerase treated PCR products (50 ng) were mixed in 10 µl and allowed for annealing at 22°C for 30 min. After heat inactivation of T4 DNA polymerase, 5 µl of annealing mix was used to transform competent DH5α cells. The transformants were screened for the presence of recombinant plasmids with KMP-11 insert by PCR under the similar conditions as mentioned before. Isolated positive clones were sequenced by DNA sequencer (ABI PRISM, model 377). Control vector without insert was generated by double restriction digest of pCMV-LIC KMP-11 at EcoR1 and Apa1 sites flanking the insert. 2 µg of the construct was initially digested with 10 U EcoR1 at 37°C in a total volume of 40 µl (500 mM Tris, pH 8.0, 100 mM MgCl₂, 500 mM NaCl) followed by 5' end-filling with Klenow fragment in presence of dNTPs in a total volume of 20 µl (0.5 mM dNTP mix, 2 U Klenow fragment) and incubated for 10 min at 37°C. After purification, the DNA was digested with Apa1 at 30°C for 1 h followed by removal of 3'-overhangs generated by Apa1 a reaction mixture containing purified DNA, 1 × T4 DNA Polymerase reaction buffer and 20 µM of each dNTPs were added along with 1 U of T4 DNA polymerase and incubated at 15°C for 30 min. After heat inactivation of T4 DNA polymerase at 75°C for 10 min, the DNA was purified as mentioned above. Finally 50 ng linear DNA was ligated by blunt-end ligation in a final volume of 50 µl (250 mM Tris-HCl pH 7.6, 50 mM MgCl₂, 5 mM ATP, 5 mM DTT, 25 % PEG 8000 solution (w/v), 2 U T4 DNA Ligase) at 14°C for 24 h. T4 DNA Ligase was inactivated by heating at 70°C for 10 min. The reaction mixture was used directly for transformation and the colonies negative for KMP-11 was checked by PCR and selected.

### 2. Bone marrow derived Dendritic Cell and Macrophage Culture:

BMDC were generated from bone marrow progenitors in presence of GM-CSF and IL-4 as previously reported (17, 18, and 19). Briefly, bone marrow cells, collected after passing of marrow from tibias and femurs of C57BU6 or BALB/c mice through nylon mesh were suspended in complete RPMI 1640 and incubated at 37°C in presence of 5% CO₂ supply for 2 h. Then 10⁶/ml non-adherent cells were reseeded in a 24 well plate in presence of rmGM-CSF (150U/ml) and rmIL-4 (75 U/ml) and cultured for 3 days in a CO₂ incubator. On day 3 non-adherent cells (2.5 x 10⁶/2ml/well) were again transferred into a six well plate supplemented with complete medium and cytokines and subsequently cultures were fed with rmGM-CSF and rmlL-4 on days 5 and 7. After 10 days, the non-adherent cells were collected and shown to have a typical DC morphology. Phenotypic characteristics of these cells were evaluated by flow cytometry. Last 14-18 h of BMDC cultute was allowed in presence of DC maturation stimulus rmTNF-α (20ng/ml). Now these BMDC were used in subsequent experiments.

BMDM were obtained by culturing 10⁶ bone-marrow cells in complete RPM11640 medium containing 0.2 ng/ml rmGM-CSF and 50 ng/ml rmM-CSF in a 6 well plate and incubated at 37°C for 24 h. After which non-adherent cells were transferred to a new well. with the same medium and cytokines and cultured for an additional 5-7 days at 37°C in a CO₂ incubator (20).

### 3. Transfection of BMDCs and BMDMs

Endotoxin-free KMP-11 plasmid DNA isolated using EndoFree^{®} Plasmid Mega Kit (Qiagen, GmbH, Hilden, Germany) according to the manufacturer's instruction was used for transfection of bone-marrow derived DC and macrophages with Effectene Reagent (Qiagen, GmbH, Hilden, Germany) according to the manufacturers' protocols. Transfection efficiency of BMDM and BMDC was estimated by counting red fluorescent cells under inverted fluorescent microscope when transfected with a red fluorescent protein carrying plasmid pDsRED (Clonetech). BMDC showed transfection efficiency < 6% while BMDM showed transfection efficiency of ∼18% (21).

### 4. Electrofusion Parameters and Hybrid cell Generation

Allogeneic BMDC were fused with pCMV-LIC/KMP-11 transfected syngeneic BMDM/BMDC by electric fusion as described previously (22, 23, 24, 25). Briefly, BMDC and pCMV-LIC/KMP-11 transfected BMDM/BMDC were washed thoroughly with sterile 0.3 M isotonic sucrose solution Immediately before fusion 2 X 10⁷/ml viable BMDCs and 2 X 10⁷/ml viable pCMV-LIC/KMP-11 transfected primary Mϕ /DC were mixed at a ratio of 1:1 to yield a density of 2 X 10⁷/ml in an inert wax coated electroplated cuvette (Bio-Rad). Inert wax was applied to one electrode of the cuvette by pitting 100-200 µl liquid wax into a horizontally kept cuvette to generate an inhomogenous electric filed. Now mixed cells suspended in isotonic sucrose solution were first di-electrophoretically aligned to bring the cells in to close contact by applying a low voltage (20 for 20 s) spatiallly nonuniform electric field in an electrofusion chamber (Custom make) followed by simultaneously applied single square pulse of 625 V/cm setting of a gene pulser (Bio-Rad) (26). After electrofusion, the cuvette was left undisturbed for 5 min before cells were kept suspended in relaxation buffer for 15 min (100mM KCl, 3mM NaCl, 1.25 mM EDTA, 10 mM HEPES, 0.5 mM ATP). Cell viability determined by dye exclusion was usually >90%.

### 5. Standardization and optimization of Electro fusion Parameters for hybrid cells generation

As a result of Brownian motion and the repellent electrostatic forces arising from the net negative charge on the outer membrane surface, cells in suspension will not come into close contact. Therefore, close membrane contact is one of the prerequisites for fusion, which is achieved by di-electrophoretic alignment and mutual interaction of cells by applying a low alignment voltage. After alignment of suspended cells in a non-uniform electric field fusion of membranes at the site of contact is achieved by applying pulse voltage of specific pulse length and fusion between numbers of cells in suspension is achieved by increasing cell density. The optimum conditions for the electric field induced fusion are achieved when the membranes of two adjacent cells flatten out on coming into contact with each other, thus forming a relatively large zone of contact by applying 20 V alignment voltage (50 V/cm) and simuftaneously applied 250 V square pulse voltage (625 V/cm) in a wax coated cuvette (0.4 cm path length) containing two different types of cells (2 x 10 ⁷/ml each) namely allogeneic BMDC and syngeneic KMP-11 transfected BMDC (**Fig. 1A & 1B**) or allogeneic BMDC (H-2^{b}) and pCMV-LIC/KMP-11 transfected syngeneic BMDM (H-2^{d}) (**Fig. 1C & 1D**).

### 6. Generation of hybrids

For electrofusion, 2 X 10⁷/ml viable allogeneic BMDC (H-2^{b}) and 2 X 10⁷/ml viable KMP-11 transfected syngeneic BMDM (H-2^{d}) suspended in isotonic sucrose (0.3 M) solution mixed at a ratio of 1:1 to yield a density of 2 X 10⁷/ml in an inert wax coated electroplated cuvette of 0.4 cm path length (Bio-Rad) were first di-electrophoretically aligned to bring the cells in to close contact by applying a low voltage (50V/cm for 20 s) spatiallly nonuniform electric field in an electrofusion chamber (custom made) followed by simultaneously applied single square pulse of 625V/cm setting of a gene pulser (Bio-Rad). After electrofusion, the cuvette was left undisturbed for 5 min before cells were kept suspended in relaxation buffer for 15 min. Cell viability was checked by dye exclusion method. To quantitate percentage of viable hybrid cell generation, pCMV-LIC/KMP-11 transfected syngeneic BMDM (H-2^{d}) were stained with membrane dye PKH26 and BMDCs from C57BL/6(H-2^{b}) were stained with intracellular dye CSFE. 2 X 10⁷/ml viable BMDCs were fused with 2 X 10⁷/ml viable KMP-11 transfected macrophages forming hybrids [Syngeneic BMDM -KMP-11 + allogeneic BMDC] or a mixture of allogeneic BMDC (H-2^{b}) and syngeneic BMDM (H-2^{d})-KMP-11 cells were analysed by flow cytometry and % of viable hybrid cell generation was calculated from double positive cells in the third quadrant of a dot plot [**figure 1E and 1F**].

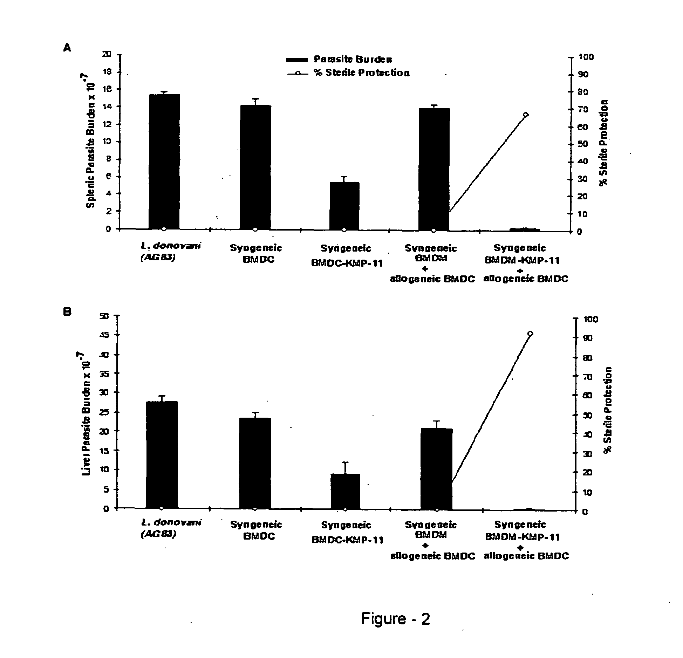

Splenic & Liver parasite burden of 105 days L. *donovani* (AG83) infected BALB/c mice received 6 i.v. injections of KMP-11 DNA transfected syngeneic BMDC (H-2^{d}) therapy or hybrid cell [Syngeneic BMDM-KMP-11 + allogeneic BMDC(H-2^{b})] therapy compared with respective untransfected syngeneic BMDC (H-2^{d}) or hybrid of untransfected syngeneic BMDM (H-2^{d}) /allogeneic BMDC (H-2^{b}) and infected control groups. For vaccination, at first, 4-6 wks old age matched BALB/c mice were challenged with AG83 (1 x 10⁶) live promastigotes through intra cardiac route and 60 days after infection mice were treated with 6 i.v. injections of 5 x 10⁵ hybrid cells or syngeneic BMDC 5 days apart. Vaccinated mice were sacrificed 15 days after last injection. Organ parasite burden was assayed by limiting dilution assay method in all cases. The results are depicted in **figure 2****.** The reciprocal of the highest dilution that was positive for parasite growth was considered to be the concentration of parasite/mg of tissue. Total organ parasite burden was calculated from spleen or liver weight. Data represent the mean ± SE for 12 animals per group.

Splenic parasite burden of 105 days AG83 infected BALB/c mice therapeutically treated with different kinds of hybrid cells or syngeneic BMDC compared with infected control groups of mice. *, p<0.0005 (Syngeneic BMDC-KMP-11 vs. AG83 challenged group.); **, p<0.05 [(Syngeneic BMDM + allogeneic BMDC) vs. AG83 challenged group] Asterisks represent level of significant variance compared with the groups mentioned [**see** **figure 2A**]

Hepatic parasite burden of 105 days AG83 infected BALB/c mice therapeutically treated with different kinds of hybrid cells or syngeneic BMDC compared with infected control groups of mice. In terms of liver parasite burden sterile protection was observed for all hybrid cells' (Syngeneic BMDM-KMP-11 + allogeneic BMDC) vaccinated mice. o, p<0.0005 (Syngeneic BMDC-KMP-11 vs. AG83Inf.); oo, p<0.05 [(Syngeneic BMDM + BMDC) vs. AG83 challenged group [**see** **figure 2B**].

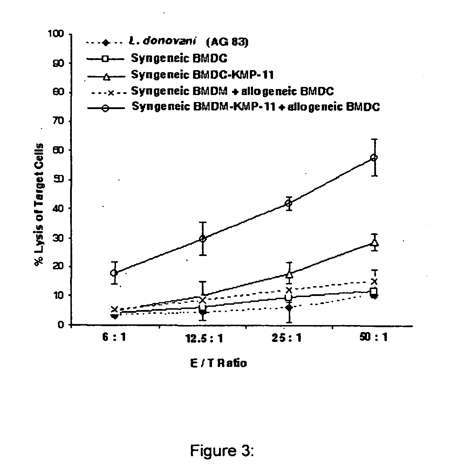

**Figure 3** represents the Anti-KMP-11 CTL response in hybrid cells or KMP-11 DNA transfected syngeneic BMDC vaccinated 105 days *L*. *donovani* post infected mice compared to respective control groups. Target cells were RAW 264.7 macrophage cell line and labeled with ⁵¹Cr after transfecting with pCMV-LIC/KMP-11 or pCMV-LIC plasmid DNA and used as target. Splenocytes (Effector) from different groups of vaccinated and control groups of mice were pulsed with SLA for 7 days. Spontaneous release was less than 15%. Results are representative of 5 individual experiments. 5 animals/group /experiment and data represent the mean ± SE. At 50:1 and 6:1 E/T ratio hybrid cell vaccinated mice showed *, p<0.0005; ** p<0.0001 respectively in comparison with respective infected control groups, while KMP-11 DNA transfected syngeneic BMDC vaccinated mice at 50:1 and 6:1 E/T ratio showed o, p<0.00001; oo, p<0.5 respectively' compare to infected control.

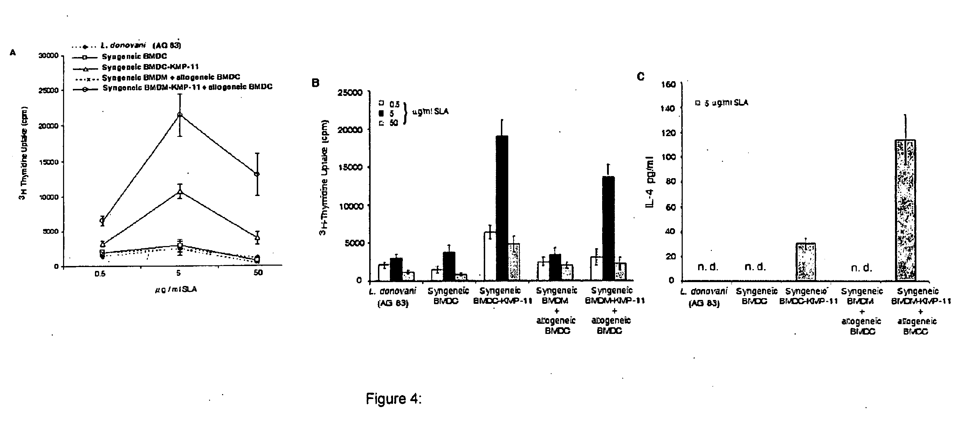

T cell proliferation, IL-2 and IL-4 assay in response to SLA stimulation between different groups of experimental mice is depicted in **figure 4****.** 24 h supernatant from SLA stimulated T cell proliferation assay was used for IL-2 functional assay and IL-4 cytokine assay. The results are representative of 3 individual experiments (n=5/ group) and data represent the mean of triplicate wells ± SE.

**Fig 4** **A** depicts the proliferative response to SLA (5 µg/ml) by splenocytes from different kinds of hybrid cells or genetically engineered syngeneic BMDC treated AG83 infected BALB/c mice compared with respective controls. Proliferation was measured by ³H-thymidine incorporation. At 5 µg/ml SLA stimulation *, p<0.0005 for hybrid (syngeneic BMDM / allogeneic BMDC) and syngeneic BMDC-KMP-11 treated group with respect to *L. donovani* (AG83) challenged group.

**Fig 4** **B** depicts the production of IL-2 by spleen cells from therapeutically vaccinated mice treated with either different kinds of hybrid cells or syngeneic BMDC compared with control groups in terms of growth of an IL-2 dependent murine cell line HT2 proportional to ³H-thymidine incorporation. At 5 µg/ml SLA stimulation **, p<0.005 for hybrid cell (syngeneic BMDM-KMP-11/ allogeneic BMDC) treated group with respect to *L. donovani* (AG83) challenged group and ***, p<0.0005 for syngeneic BMDC-KMP-11 with respect to *L. donovani* AG83 challenged group.

**Fig 4** **C** depicts the quantitation of IL-4 in the supernatant of 24 h SLA (5 µg/ml) stimulated splenocytes from different groups of treated and untreated BALB/c mice by ELISA.

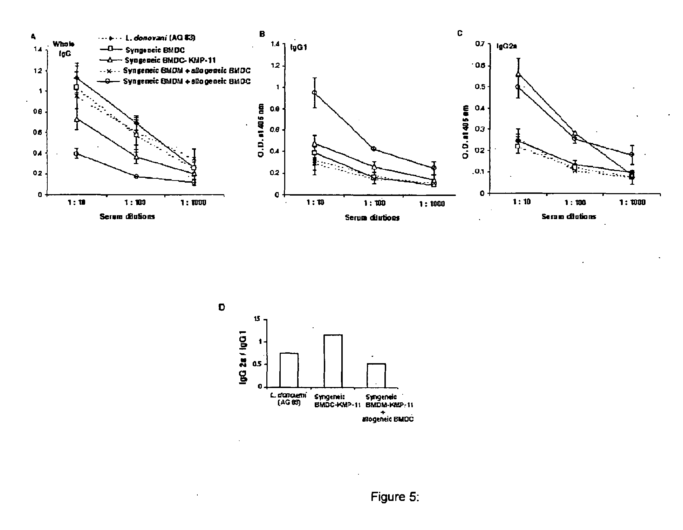

Anti-KMP-11 whole IgG, IgG1 and IgG2 Ab titers in the sera of hybrid cells or KMP-11 DNA transfected syngeneic BMDC treated 105 days AG83 infected BALB/c mice (n=5/group) are represented by **figure 5A, 5B & 5C**. The results are representative of 3 experiments and data represent the mean ± SE. In case of whole IgG *, p<0.0005 hybrid cell treated (Syngeneic BMDM-KMP-11 / allogeneic BMDC) vs. *L. donovani* challenged group at 1/100 serum dilution] and **, p<0.05 (KMP-11 DNA transfected syngeneic BMDC vs. *L. donovani* challenged group at 1/100 serum dilution); for IgG1 †, p<0.5 hybrid cell treated (Syngeneic BMDM-KMP-11 / allogeneic BMDC) vs. *L. donovani* challenged group at 1/100 serum dilution] and ††, p<0.5 (KMP-11 DNA transfected syngeneic BMDC vs. *L. donovani* challenged group at 1/100 serum dilution) which are not statistically significant. In case of IgG2a °, p<0.005 [(Syngeneic BMDM-KMP-11 + allogeneic BMDC) vs. *L. donovani* challenged group at 1/100 serum dilution] and °°, p<0.0005 (KMP-11 DNA transfected syngeneic BMDC vs. *L. donovani* challenged group at 1/100 serum dilution).

Figure 5D represents the ratio of anti-KMP-11 IgG2a/IgG1 titers in the sera from different infected and therapeutically vaccinated BALB/c mice.

***The following examples are given by way of illustration* of *the present invention and therefore should not be construed to limit the scope of the present invention.***

### Example 1

### FACS analysis and fluorescent microscopy to determine fusion efficiency:

To assess the fusion efficiency the partner cells were stained separately with different fluorescent dyes. In our case before electrofusion allogeneic BMDC were labeled with intracellular dye CSFE (Molecular Probes) and pCMV-LIC/KMP-11 transfected syngeneic BMDM cells were stained with a membrane dye PKH26 (Sigma, St. Louis, MO) according to manufacturer's instructions and subjected to above electrofusion procedure. The fusion efficiencies were >30% as evident from double stained cells detected by flow cytometry and fluorescent microscopy when superimposed (27).

### Example 2

### Therapeutic Vaccination Protocol:

For vaccination, two months *L. donovani,* AG83 infected BALB/c [*L. donovani,* (AG 83)] were divided into several groups receiving at least 6 i. v. injections of 5 X 10⁵ cells/animal/100µl PBS 5 days apart: one group was vaccinated with syngeneic BMDCs transfecfed with KMP-11 containing pCMV-LIC mammalian expression vector [Syngeneic BMDC-KMP-11] or untransfected syngeneic BMDC [Syngeneic BMDC]. Of other two groups of 2 mo A83 infected mice one was vaccinated with hybrids of pCMV-LIC-KMP-11 transfected syngeneic BMDM and bone marrow derived allogeneic BMDC (C57BU6) [Syngeneic BMDM-KMP-11 + allogeneic BMDC] and other groups was treated with hybrids of untransfected syngeneic BMDM and allogeneic BMDC [Syngeneic BMDM + allogeneic BMDC]. We also had taken into consideration of therapeutic vaccination with syngeneic BMDC transfected with blank-vector construct not harbouring KMP-11 gene (pCMV-LIC) and hybrids of pCMV-LIC transfected syngeneic BMDC and allogeneic BMDC (data not shown). Mice in all groups were sacrificed 15 days after receiving the last vaccine (105 days post infection) for determination of parasite burden and subsequent experiments.

### Therapeutic vaccination with KMP-11 transfected BMDM/Allogeneic BMDC hybrid cells clears both splenic and hepatic parasite burden

**The virulent L. *donovani* strain-AG83 (MHOM/IN/1983/AG83)** (Originally isolated from a Kala-azar patient, this strain of *Leishmania donovani* parasite is routinely maintained in this institute. MHOM is a standard World Health Organization nomenclature : IN stands for country of origin "India"; 1983 stands for the Year of Discovery; AG83 stands for the strain-specific name. The strain is available with the inventors at IICB, Kolkata, India on demand by any interested person/organization for academic and research purposes) used in this study (31) has a different kinetics of infection than the Sudanese (LV9, LV82) and Ethiopian *L. donovani* used in other studies (32, 33, 34). After AG83 challenge, an exponential rise in both splenic and hepatic parasite burden is observed, at least, 5 mo post-infection in BALB/c mice model. Therefore we took a late stage infected mice (60 days infected model), to study the efficacy of therapy in advanced state of the disease. Devising out experiment was based on this conceptual basis of combining the association of immunodominant KMP-11 with syngeneic class I in combination with allogeneic MHC class II bearing DC in a single hybrid cell entity expected to bring about a vigorous T-T collaboration and induction of strong antigen specific CTLs directed against KMP-11 of *Leishmania.* To study the therapeutic efficacy of hybrid cell vaccine over genetically engineered BMDC vaccine, in 60 days *L. donovani* infected BALB/c mouse model, KMP-11 DNA transfected syngeneic BMDC or KMP-11 DNA transfected BMDM (H-2^{d}) electrofused with allogenic BMDC (H-2^{b}) were injected 6 times intravenously every 5 days. 15 days after the last injection, mice were sacrificed for subsequent experiments. Remarkably in all infected BALB/c mice receiving hybrid cell [BMDM (H-2^{d})-KMP-11 + BMDC (H-2^{b})] vaccine (5 x 10⁵cells/ injection) there was complete absence of amastigotes in the impressions of stamp smears of transverse sections of spleens and livers when observed under light microscopy (data not shown). Consistently spleen cell culture of > 66% of mice treated with hybrids of KMP-11 DNA transfected syngeneic BMDM (H-2^{d})/allogeneic-BMDC (H-2^{b}) showed complete absence of promastigotes in the serially diluted culture (only 8 out of 24 mice showed > 98% reduction of splenic parasite burden by serial dilution assay) (Figure 2A). Serial dilution assay confirmed complete clearance of hepatic parasite burden of >90% of the hybrid cell vaccinated mice (3 out of 24 mice showed ∼99% reduction in liver parasite burden as determined by standard serial dilution assay) (Figure 2B). Whereas pCMV/LIC-KMP-11 transfected syngeneic BMDC treated mice could only confer around 64% clearance of splenic parasite burden (p<0.0005) and about 67% reduction of liver parasite burden (p<0.0005). Both the control groups of untransfected syngeneic BMDC (p<0.5) and hybrids of untransfected syngeneic BMDM/Allogeneic-BMDC (p>0.05) could not induce any significant protection in organ parasite burden.

### Example 3

### Determination of splenic and hepatic parasite burden by serial dilution method:

The parasite burden was quantified in spleen and liver tissue by serial dilution assay as described previously (28). Weighed piece of spleen or liver from experimental BALB/c mice was first homogenized between two sterile frosted glass slides in complete M199 medium and diluted with the same medium to a final concentration of 1 mg/ml 10 fold serial dilutions of the homogenized tissue suspensions were then plated in 96-well plate and incubated at 22°C for 2-3 wks. Wells were examined for viable and motile promastigotes every 3 days interval and reciprocal of the highest dilution that was positive for parasites was considered to be the parasite concentration per mg tissue. The total organ parasite burden was calculated using the weights of respective organs.

### Example 4

### Electrofusion Parameters and Hybrid cell Generation:

BMDC were fused with pCMV-LIC/KMP-11 transfected P388D1 by electric fusion as described previously. Briefly, BMDC and pCMV-LIC/KMP-11 transfected P388D1 cells were washed thoroughly with sterile 0.3 M isotonic sucrose/NaCl solution (molecular formulation of isotonic sucrose/ NaCl solution was done from Bergey's manual of chemical solutions). Immediately before fusion 2 X 10⁷/ml viable BMDC and 2 X 10⁷/ml viable pCMV-LIC/KMP-11 transfected P388D1 cells were mixed at a ratio of 1:1 to yield a density of 2 X 10⁷/ml in an inert wax coated electroplated cuvette (Bio-Rad). Inert wax was applied to one electrode of the cuvette by pitting 100-200 µl liquid wax into a horizontally kept cuvette to generate an inhomogenous electric filed. Now mixed cells suspended in isotonic sucrose/ NaCl solution were first di-electrophoretically aligned to bring the cells in to close contact by applying a low voltage (20V for 20S) spatially nonuniform electric field in an electrofusion chamber followed by simultaneously applied single square pulse of 625V/cm setting of a gene pulser (Bio-Rad). After electrofusion, the cuvette was left undisturbed for 5 min before cells were kept suspended in relaxation buffer for 15 min. Cell viability determined by dye exclusion was usually >90%. The fused cells were then washed with PBS and irradiated with 100 Gy and used for vaccination directly.

### Square wave pulse at 625V/cm at an alignment voltage of 20V (50 V/cm) results in greater than 30% viable, fused KMP-11 transfected BMDM (H-2^{d})/BMDC (H-2^{b}) hybrid cells

Electrofusion, a technique most suitably used for fusing two cell types, excludes undesirable fusogenic chemicals and viruses, outmatching other means of hybrid cell generation in its efficiency. Initial standardization of electrofusion were obtained by a series of optimization conditions subjecting the partnering cells (syngeneic BMDM with allogeneic BMDC and syngeneic BMDC with allogeneic BMDC) to a range of alignment voltage (10-40V) and pulse voltage (100-400V) and corresponding viable hybrid cell generation was noted (Figure 1 A, B, C & D). Energy transfer by square wave pulses of same width and height are much more efficient than exponential wave pulses.

As cell viability and efficient membrane fusion are the two important aspects of hybrid cell formation, decrease of pulse height in accordance with increase of pulse width suggested us to choose square wave pulse for complete fusion of membranes at their site of contact, where cells were aligned in a di-electrophoretic field of wax coated cuvette (0.4cm path length) (44). For both sets of partnering cells participating in fusion process (syngeneic BMDM-KMP/allogeneic BMDC & syngeneic BMDC-KMP/allogeneic BMDC), results showed that although at higher greater alignment voltage (>25 V), the percentage of hybrid formation increased, the resultant viability of the cells decreased considerably keeping the pulse voltage constant. Similarly at constant alignment voltage, viability is rapidly decreased at higher pulse voltage despite greater fusion efficiency (Figure 1 A, B, C & D). In case of fusion of syngeneic BMDC with allogeneic BMDC, DC-DC hybrid formation never exceeded 15.5% (FACS data not shown) with a corresponding viability of 40% (Figure 1A & B). Overall viability of DC-DC hybrids never exceeded 43% at any combination of alignment voltage and pulse volt. In case of electrofusion of syngeneic BMDM with allogeneic BMDC at a constant optimized alignment voltage of 20 V, a high yield of fused hybrid cells was generated by a square pulse above 225 V reaching the maximum yield at 300 V along with a steady decline in viability of hybrid cells. Thus >85% viable fused cells (KMP-11 transfected BMDM/allogeneic BMDC hybrid) were obtained at an alignment voltage of 20 V (50 V/cm) and a square wave pulse of 250 V (1000 V/cm).

Fusion of allogeneic DC with KMP-11 transfected BMDM when compared to untransfected BMDM did not show significant variability in terms of hybrid formation or viability at these optimized parameters. Thus for therapeutic vaccination purpose hybrids of BMBM-BMDC were used for their high percentage of fusion (> 30%) and corresponding high viability (> 85%). Analysis of hybrid cells used for vaccination purpose was performed by FACS analysis and further verified by fluorescent microscopy. Transfected BMDM cells, on one hand, were stained by red fluorescent dye PKH26 and, on the other hand, allogeneic BMDC were stained by the intracellular green fluorescent dye, CFSE. After electrofusion, flow cytometry was done to monitor dually fluorescent events. Mixed population of cells without electrofusion was taken as a control that recorded few dually fluorescent events (Figure 1 E & F). As FACS data in some instances might be misleading due the presence of cellular aggregates, we also observed the electrofused cells under fluorescent microscopy to determine true membrane fusion events. Thus the hybrid cells were visualized separately under red and green filter separately and a two-color overly was performed to observe hybrid cells staining for both red (PKH 26) and green (CFSE) (Figure 1 G, H & I):

### Example 5

### T Cell Proliferation Assay:

T cell proliferation assay was performed as described (29). Single cell suspension of splenocytes from different experimental groups of BALB/c mice 95 days post-infection with AG83 were prepared after Ficoll density gradient centrifugation and then suspended in complete RPMI 1640. Cells were plated in triplicate at 10⁵ cell/well concentrations in 96-well plates and allowed to proliferate for 3 days .at 37°C in 5% CO2 incubator in presence of different SLA concentrations (0.5, 5, 50 µg/ml) and also without SLA. 18 h before they were harvested, cells were pulsed with 1 µCi (6.7 Ci/mmole) [³H] thymidine / well. ³H-thymidine uptake, as an index of proliferation, was measured by Liquid Scintillation Counter (TRI CARB 2100TR, Packard).

### SLA induces signifcantly higher T cell proliferation with high IL-2 & IL-4 production in therapeutically hybrid cell treated mice

Cell mediated immune response is impaired in VL patients as well as in experimental models hallmarked by T cell anergy specific to *Leishmania* Ag (30). As hybrid cell vaccination could reverse impaired cell-mediated immune response by inducing strong CTL generation, we were interested to observe effect of hybrid cell therapy in antigen-specific T cell proliferative response. To assess the T lymphocyte response to SLA in untreated and treated mice groups, splenocytes were stimulated with various concentrations of SLA. KMP-11 DNA transfected syngeneic BMDC treated mice showed 4.3-fold enhancement of lymphocyte proliferation (p<0.0005), hybrid cell treated mice showed 8.84-fold enhancement of proliferative (p<0.0005) response at 5µg/ml SLA concentration with respect to infected group (Figure 4 A). Thus hybrid cell vaccinated mice showed about 2 fold more proliferation index (p<0.05) with respect to KMP-11 DNA transfected syngeneic BMDC treated mice. As IL-2 is known to have leishmanicidal activity in the experimental BALB/c mice model for VL (36), IL-2 production was assayed in 24 h SLA simulated supernatant of splenocytes from vaccinated mice as an index of ³H thymidine uptake by HT2 cells bearing high affinity receptors for IL-2/IL-4 ( 37, 38). Hybrid cell treated mice showed about 5.11 folds more IL-2 generation (p<0.005) whereas syngeneic KMP-11 DNA transfected BMDM treated mice showed about 6.19 fold more IL-2 generation (p<0.0005) compared to the infected control group of mice at 5 µg/ml SLA concentration (Figure 4 B). Surprisingly, despite partial protection conferred by the KMP-11 DNA transfected BMDC treated group, IL-2 generation was 1.21 fold more (p<0.5 at 5 µg/ml SLA), 1.61 fold more (p<0.5 at 0.5 µg/ml SLA) and 1.81 fold more (p<0.5 at 50 µg/ml SLA) compared to hybrid cell treated group. IL-2R 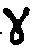 is demonstrated as a functional component of the IL-4 receptor, on the basis of chemical cross-linking data, the ability of IL-2R gamma to augment IL-4 binding affinity, (39). Due to comparable amount of IL-2 generation despite conferring partial and complete protection in the transfected DC and hybrid cell treated groups respectively despite 2 fold more T cell proliferation in the latter, we looked into the role of IL-4 in antigen-specific lymphocyte proliferation assay due its established role as a T cell growth factor (40) and its role in the proliferation of IL-4 receptor bearing HT2 cells. IL-4 was measured by ELISA at the same time point of measurement of IL-2 (24 h supernatant of the SLA restimulated splenocytes) where we found a marked enhancement of IL-4 in the supernatant of the hybrid cell treated mice splenocyte (114 pg/m) ± 20.5) compared to that of KMP-11 DNA transfected syngeneic BMDC treated group (24.8 pg/ml ±6.43) (Figure 4 C). IL-4 could not be detected in the splenocytes of infected and other control groups of mouse. This indicated towards IL-2 and IL-4 generation as a result of curative immune response in therapeutically treated mice. (41).

### Example 6

### IL-2 Assay

Splenocytes (5x10⁵ cells/well) of BALB/c mice derived from different groups of experimental animals 105 days AG83 post-infected were stimulated for 24 h with SLA (0.5, 5, 50 µg/ml) or without SLA in complete RPMI 1640 medium in 5% CO₂ incubator at 37°C. The culture supernatants were analyzed for the presence of IL-2 by proliferation of IL-2 dependent murine cell line (HT-2), and extent of proliferation was measured by ³H-thymidine uptake. 10⁴ HT-2 cells/well were incubated with 100 µl of culture supernatant for 48 h. The cells were then pulsed with 1µCi of ³H-thymidine (6.7 Ci/mmole) for 18 h (29). Incorporation of radioactive thymidine was assessed by Liquid Scintillation Counter (TRI CARB 2100TR, Packard).

### Example 7

### Cytotoxic T lymphocyte Assay

Non-adherent splenocytes from different groups of infected and 2 mo AG83 infected BALB/c mice received therapeutic treatment were stimulated with SLA for 7 days followed by incubation with ⁵¹Cr-labeled targets (RAW 264.7 transfected either with pCMV-LIC, pCMV-LIC KMP-11 or pEGFP-N1) in round bottom 96-well plates (200 µl) at different (6:1, 12.5:1, 25:1, 50:1) effector: target (E/T) ratio for 4 h. Target cells (10⁶) were labeled with 100 µCi Na₂CrO₄ (2.5 mCi/38.4 µg/ml, BARC, Mumbai, India) for 1 h at 37°C in 5% CO₂ incubator and washed several times till no γ irradiation count were detected in the supernatant. After 4 h incubation, 100 µl of culture supernatant was collected and counted in triplicates in Liquid Scintillation Counter (TRI CARB 2100TR, Packard). Specific lysis was calculated according to the formula: % Specific Lysis = (Sample -Spontaneous release) / (Maximum release-Spontaneous release) X 100.

### Therapeutic vaccination with Hybrid Cells induces high anti-KMP-11 cytotoxic T lymphocyte response in 2 months L. donovani infected BALB/c mice

Induction of parasite antigen specific CTL response is one of important objectives of hybrid cell vaccination approach as it can potentially induce both Th and CTL response by allogeneic MHC molecules (H-2^{b}) presented on one of the partners of hybrid cell. Thus we proceeded to measure KMP-11 specific CTL generation in therapeutically cured group of hybrid cell treated mice. Abrogation of protective efficacy of LACK-DNA vaccination in murine CL model as a consequence of CD8⁺T cell depletion is suggestive of vaccination-induced dominant role of CD8⁺ T cell mediated protective immune response although the role of CD8+ restricted CTL has not been exclusively implicated ( 35). In our case SLA stimulated splenocytes for 7 days from mice therapeutically treated with hybrid cells [BMDM (H2^{d})-KMP-11 + BMDC (H-2^{b})] (105 days post-infection) showed a 58% lysis of ⁵¹Cr-labeled KMP-11 transfected syngeneic macrophages at 50:1 E/T ratio (p<0.0005) and 18% lysis of ⁵¹Cr- labeled targets at 6:1 E/T ratio (p<0.05). Whereas, KMP-11 DNA transfected BMDC treated mice showed a maximal lysis of 29% at 50:1 E/T ratio (p<0.005) (Figure 3). The infected and control group of mice showed less than 11% lysis of labeled targets at 50:1 E/T ratio and did not differ significantly between infected and hybrids of untransfected syngeneic BMDM and allogeneic BMDC treated control groups (p<0.5 at 50:1 E/T ratio) suggestive of impaired CTL response due to *L. donovani* infection in BALB/c model. The specificity of lysis was further corroborated by the failure of the non-adherent splenic T cells to lyse syngeneic macrophages transfected with Enhanced Green Fluorescent Protein (EGFP) expressing pEFGP-N1 construct (data not shown). Role of allogeneic MHC in generation vigorous CTL response was also supported by the observation that mice treated with hybrid cells showed statistically significant level of CTL generation at 50:1 (p<0.005) 25:1 (p < 0.0005), 12.5:1 (p<0.005) and 6:1 (p<0.05) E/T ratio than mice therapeutically treated KMP-11 DNA transfected syngeneic BMDC.

### Example 8

### Measurement of KMP-11 specific serum-Ig titers in therapeutically vaccinated mice:

KMP-11 protein under native condition was purified from **pQE-30 plasmid** [like all other plasmid vectors (cloning vectors) pQE-30 is commercially available] containing KMP-11 ORF of L. infantum aligned in frame with His-tag as described previously (30). Serum samples from different groups of BALB/c mice (n=5/group) were obtained 105 days after parasite challenge and were analyzed for the KMP-11 specific Ab titer. 96-well ELISA plates (Nunc, Wiesbaden, Germany) were coated with KMP-11 (2 µg/ml) in PBS overnight at 4°C. Plates were blocked with 5% FCS in PBS at room temperature for 1 h to prevent nonspecific binding. Sera from different groups of hamsters was added at various dilutions (1:10, 1:100, and 1:1000) and incubated for 2 h at room temperature. AKP conjugated rat anti-mouse IgG1 mAb, rat anti-mouse IgG2a mAb was added for 1 h at room temperature followed by another 30 min incubation with substrate reagent pNPP (3 mM) as alkaline phosphatase substrate in buffer (50mM Tris pH 8.5, 1 mM MgCl₂) followed by stopping of reaction with 3 M NaOH and absorbance was read on ELISA plate reader (Multiskan MS, Labsystem) at 405 nm.

Hybrid cell vaccination therapy induces a Th2 type humoral immune response associated with reduced antigen- specific whole IgG production but increased antigen-specific IgG1 and IgG2a Although successful vaccination approach emphasizes on the successful antigen targeting to MHC class I processing pathway and eliciting specific cytokines production that favour CD8⁺ T cell priming and differentiation, recent studies showed the importance natural antibodies in infective process (42). Moreover, Antibody class switching is a reliable accessory marker of Th1 and Th2 differentiation of naïve CD4⁺ T cells. Therefore, we analyzed whole IgG profile along with its isotypes (IgG1 and IgG2a) in response to KMP-11 protein. We observed significant decrease in anti-KMP-11 specific whole IgG titre in both hybrid cell (syngeneic BMDM-KMP-11 / allogenic BMDC) treated mice (p<0.0005) and KMP-11 DNA transfected syngeneic BMDC treated mice (p<0.05) compared to *L*. *donovani* infected BALB/c mice (Figure 5 A). Nevertheless with respect to KMP-11 DNA transfected syngeneic BMDC treated group hybrid cell vaccinated mice (p<0.5) did not show significant decrease in whole anti-KMP-11 IgG serum titre. Hence, our finding lays credence to previous observation that in both experimental CL and human VL model where high anti-leishmanial IgG titre is significantly decreased with disease resolution (43).

Surprisingly, both anti-KMP-11 IgG1 and IgG2a titre at 1/100 serum dilution was significantly enhanced in hybrid BMDC treated mice (p<0.05 and p<0.005 respectively) (Figure 5 B & C). Whereas in response to KMP-11 protein only IgG2a (p<0.0005) but not IgG1 (p<0.5) was significantly enhanced in partially curative response of syngeneic KMP-11DNA transfected BMDC treated mice compared to infected controls. With respect to KMP-11 transfected syngeneic BMDC, hybrid cell vaccinated mice showed statistically significant level of anti-KMP-11 IgG1 response (p<0.05) but not IgG2a response (p=0.6601). Consistent with the idea of Ab class switching as a surrogate marker for helper CD4⁺ T cell differentiation, serum from hybrid cell vaccinated mice contained high levels of KMP-11 specific IgG1 and low levels of IgG2a Ab and exhibited lowest IgG2a/IgG1 ratio (0.603), which is 1.25 times lower than that of infected control group (0.751) and 1.8 times lower than therapeutically syngeneic KMP-11 transfected BMDC treated group (1.08) (Figure 5 D). This indicated that a dominant Th2 response with high IgG2a/IgG1 ratio might not always associated with curative in experimental VL model in BALB/c mice.

The mammal for all the experiments of the present invention is a mouse and has the potential for trials of therapeutic application to primates and humans in future.

### References :

1. Liew F.Y., and C. A. O'Donnell. 1993. Immunology of leishmaniasis. Adv. Parasito/. 32: 161- 259.
2. Bottrel, R. L., W. O. Dutra, F. A. Martins, B. Gontijo, E. Carvalho, M. Barral-Netto, A. Barral, R. P. Almeida, W. Mayrink, R. Locksley, and K. J. Gollob. 2001. Flow cytometric determination of cellular sources and frequencies of key cytokine-producing lymphocytes directed against recombinant LACK and soluble Leishmania antigen in human cutaneous leishmaniasis. Infect. Immun. 69: 3232-3239.
3. Melby, P. C., V. V. Tryon, B. Chandrasekar, and G. L. Freeman. 1998. Cloning of Syrian hamster (Mesocricetus auratus) cytokine cDNAs and analysis of cytokine mRNA expression in experimental visceral leishmaniasis. Infect. Immun.66:2135.
4. Jardim, A., S. Hanson, B. Ullman, W. D. McCubbin, C. M. Kay, and R. W. Olafson. 1995. Cloning and structure-function analysis of the Leishmania donovani kinetoplastid membrane protein-11. Biochem. J. 305:315.
5. Jardim, A., V. Funk, R. M. Caprioli, and R. W: Olafson. 1995. Isolation and structural characterization of the Leishmania donovani kinetoplastid membrane protein-11, a major immunoreactive membrane glycoprotein. Biochem. J. 305:307.
6. Berberich, C., G. Machado, G. Morales, G. Carrillo, A. Jimenez-Ruiz, and C. Alonso. 1998. The expression of the Leishmania infantum KMP-11 protein is developmentally regulated and stage specific. Biochim. Biophys. Acta. 1442:230.
7. Kurtzhals, J. A., A. S. Hey, A. Jardim, M. Kemp, K. U. Schaefer, E. O. Odera, C. B. Christensen, J. I. Githure, R. W. Olafson, T. G. Theander, et al. 1994. Dichotomy of the human T cell response to Leishmania antigens. II. Absent or Th2-like response to gp63 and Th1-like response to lipophosphoglycan-associated protein in cells from cured visceral leishmaniasis patients. Clin. Exp. Immunol.96: 416.
8. Trefzer U, Weingart G, Chen Y, Herberth G, Adrian K, Winter H, Audring H, Guo Y, Sterry W, Walden P. Hybrid cell vaccination for cancer immune therapy: first clinical trial with metastatic melanoma. Int J Cancer. 2000 Mar 1; 85(5):618-26.
9. Carvalho, E. M., R. S. Teixeira, and W. D. Johnson, Jr. 1981. Cell-mediated immunity in American visceral leishmaniasis: reversible immunosuppression during acute infection. Infect. Immun. 33:498*.*
10. Carvalho, E. M., R. Badaro, S. G. Reed, T. C. Jones, and W. D. Johnson, Jr. 1985. Absence of g interferon and interleukin 2 productions during active visceral leishmaniasis. J. Clin. Invest. 76:2066*.*
11. Pintado, V., P. Martin-Rabadan, M. L. Rivera, S. Moreno, and E. Bouza. 2001.Visceral leishmaniasis in human immunodeficiency virus (HIV)-infected and non-HIV-infected patients. A comparative study. Medicine (Baltimore); 80:54.
12. United Nations Development Program/World Bank/World Health Organization 1989. The Leishmaniasis. WHO Special Program for Research and Training in Tropical Diseases. Ninth program Report. Tropical diseases: progress in international research, 1987-1988.
13. Melby, P. C., G. B. Ogden, H. A. Flores, W. Zhao, C. Geldmacher, N. M. Biediger, S. K. Ahuja, J. Uranga, and M. Melendez. 2000. Identification of vaccine candidate for experimental visceral leishmaniasis by immunization with sequential fraction of a cDNA expression library. Infect. Immun. 68: 5595.
14. Kafetzis, D. A., and H. C. Maltezou. 2002. Visceral leishmaniasis in paediatrics. Curr. Opin. Infect. Dis. 15:289.
15. Murray, H. W. 2001.Clinical and experimental advances in treatment of visceral leishmaniasis. Antimicrob. Agents Chemother. 45:2185.
16. Xu, D., and F.Y. Liew. 1995. Protection against leishmaniasis by injection of DNA encoding a major surface glycoprotein, gp63, of L. major. Immunology 84:173.
17. Ramirez-Pineda JR, Frohlich A, Berberich C, Moll H. Dendritic cells (DC) activated by CpG DNA ex vivo are potent inducers of host resistance to an intracellular pathogen that is independent of IL-12 derived from the immunizing DC. J Immunol. 2004 May 15; 172(10):6281-9.
18. Ghosh M, Pal C, Ray M, Maitra S, Mandal L, Bandyopadhyay S. Dendritic cell-based immunotherapy combined with antimony-based chemotherapy cures established murine visceral leishmaniasis. J Immunol. 2003 Jun 1; 170(11):5625-9.
19. Lutz MB, Kukutsch N, Ogilvie AL, Rossner S, Koch F, Romani N, Schuler G. An advanced culture method for generating large quantities of highly pure dendritic cells from mouse bone marrow. J Immunol Methods. 1999 Feb 1; 223(1):77-92.
20. Li Y, Chen B. Differential regulation of fyn-associated protein tyrosine kinase activity by macrophage colony-stimulating factor (M-CSF) and granulocyte-macrophage colony-stimulating factor (GM-CSF). J Leukoc Biol. 1995 Mar; 57(3):484-90.
21. Chaudhry A, Das SR, Hussain A, Mayor S, George A, Bal V. Jameel S, Rath S. The Nef Protein of HIV-1 Induces Loss of Cell Surface Costimulatory Molecules CD80 and CD86 in APCs. J. Immunol. 2005, 175: 4566-4674.
22. Scott-Taylor TH, Pettengell R, Clarke I, Stuhler G, La Barthe MC, Walden P, Dalgleish AG. Human tumour and dendritic cell hybrids generated by electrofusion: potential for cancer vaccines. Biochim Biophys Acta. 2000 Mar 17; 1500(3):265-79.
23. Trefzer U, Walden P. Hybrid-cell vaccines for cancer immune therapy. Mol Biotechnol. 2003 Sep; 25(1):63-9.
24. Trevor KT, Cover C, Ruiz YW, Akporiaye ET, Hersh EM, Landais D, Taylor RR, King AD, Walters RE. Generation of dendritic cell-tumor cell hybrids by electrofusion for clinical vaccine application. Cancer Immunol Immunother. 2004 Aug; 53(8):705-14. Epub 2004 Mar 26.
25. Haenssle HA, Krause SW, Emmert S, Zutt M, Kretschmer L, Schmidberger H, Andreesen R, Soruri A. Hybrid cell vaccination in metastatic melanoma: clinical and immunologic results of a phase I/II study. J Immunother. 2004 Mar-Apr; 27(2):147-55.
26. Zimmermann U, Vienken J. Electric field-induced cell-to-cell fusion. J Membr Biol. 1982; 67(3):165-82.
27. Parkhurst MR, DePan C, Riley JP, Rosenberg SA, Shu S. Hybrids of dendritic cells and tumor cells generated by electrofusion simultaneously present immunodominant epitopes from multiple human tumor-associated antigens in the context of MHC class I I and class II molecules. J Immunol. 2003 May 15; 170(10):5317-25.
28. Melby PC, Chandrasekar B, Zhao W, Coe JE. The hamster as a model of human visceral leishmaniasis: progressive disease and impaired generation of nitric oxide in the face of a prominent Th1-like cytokine response. J Immunol. 2001 Feb 1;166(3):1912-20.
29. Roy S, Scherer MT, Briner TJ, Smith JA, Gefter ML. Murine MHC polymorphism and T cell specificities. Science. 1989 May 5; 244(4904):572-5.
30. Basu R, Bhaumik S, Basu JM, Naskar K, De T, Roy S. Kinetoplastid membrane protein-11 DNA vaccination induces complete protection against both pentavalent antimonial-sensitive and -resistant strains of Leishmania donovani that correlates with inducible nitric oxide synthase activity and IL-4 generation: evidence for mixed Th1- and Th2-like responses in visceral leishmaniasis. J Immunol. 2005 Jun 1; 174(11):7160-71.
31. Basak SK, Saha B, Bhattacharya A, Roy S. Immunobiological studies on experimental visceral leishmaniasis. II. Adherent cell-mediated down-regulation of delayed-type hypersensitivity response and up-regulation of B cell activation. Eur J Immunol. 1992 Aug; 22(8):2041-5.
32. Alexander J, Carter KC, Al-Fasi N, Satoskar A, Brombacher F. Endogenous IL-4 is necessary for effective drug therapy against visceral leishmaniasis. Eur J Immunol. 2000 Oct; 30(10):2935-43.
33. Stager S, Smith DF, Kaye PM. Immunization with a recombinant stage-regulated surface protein from Leishmania donovani induces protection against visceral leishmaniasis. J Immunol. 2000 Dec 15; 165(12):7064-71.
34. Kaye PM, Gorak P, Murphy M, Ross S. Strategies for immune intervention in visceral leishmaniasis. Ann Trop Med Parasitol. 1995 Dec;89 Suppl 1:75-81. Review.
35. Gurunathan S, Sacks DL, Brown DR, Reiner SL, Charest H, Glaichenhaus N, Seder RA. Vaccination with DNA encoding the immunodominant LACK parasite antigen confers protective immunity to mice infected with Leishmania major.J Exp Med. 1997 Oct 6; 186(7):1137-47.
36. Murray HW, Miralles GD, Stoeckle MY, McDermott DF. Role and effect of IL-2 in experimental visceral leishmaniasis. J Immunol. 1993 Jul 15; 151(2):929-38.
37. Kupper T, Horowitz M, Lee F, Robb R, Flood PM. Autocrine growth of T cells independent of interleukin 2: identification of interleukin 4 (IL 4, BSF-1) as an autocrine growth factor for a cloned antigen-specific helper T cell. J Immunol. 1987 Jun 15; 138(12):4280-7.
38. Kupper T, Flood P, Coleman D, Horowitz M. Growth of an interleukin 2/interleukin 4-dependent T cell line induced by granulocyte-macrophage colony-stimulating factor (GM-CSF). J Immunol. 1987 Jun 15; 138(12):4288-92.
39. Russell SM, Keegan AD, Harada N, Nakamura Y, Noguchi M, Leland P, Friedmann MC, Miyajima A, Puri RK, Paul WE, et al. Interleukin-2 receptor gamma chain: a functional component of the interleukin-4 receptor. Science. 1993 Dec 17; 262(5141):1880-3.
40. Locksley RM, Heinzel FP, Holaday BJ, Mutha SS, Reiner SL, Sadick MD. Induction of Th1 and Th2 CD4+ subsets during murine Leishmania major infection. Res Immunol. 1991 Jan; 142(1):28-32.
41. Satoskar A, Bluethmann H, Alexander J. Disruption of the murine interleukin-4 gene inhibits disease progression during Leishmania mexicana infection but does not increase control of Leishmania donovani infection. Infect Immun. 1995 Dec; 63(12):4894-9.
42. Stager S, Alexander J, Kirby AC, Botto M, Rooijen NV, Smith DF, Brombacher F, Kaye PM. Natural antibodies and complement are endogenous adjuvants for vaccine-induced CD8+ T-cell responses. Nat Med. 2003 Oct;9(10):1287-92.
43. Miles SA, Conrad SM, Alves RG, Jeronimo SM, Mosser DM. A role for IgG immune complexes during infection with the intracellular pathogen Leishmania. J Exp Med. 2005 Mar 7; 201(5):747-54.

## Claims

1. A hybrid cell vaccine against leishmaniasis comprising syngeneic macrophage(s) with an immuno-dominant *Leishmania* antigen Kinetoplastid Membrane Protein-11 electrofused with allogenic dendritic cell(s).

2. A hybrid cell vaccine according to claim 1, wherein the macrophage is obtained from BALB/c mice by known methods.

3. A hybrid cell vaccine according to claim 1 or claim 2, wherein the strain of *Leishmania* is *L. donovani* from which *Leishmania* antigen Kinetoplastid Membrane Protein-11 gene is obtained and the antigen comprises an immunodominant cell surface protein of *Leishmania* Kinetoplastid Membrane Protein-11.

4. A hybrid cell vaccine according to any one of claims 1 to 3, wherein the allogenic dendritic cell is obtained from bone marrow progenitors or Bone Marrow Derived Dendritic Cells (BMDC) obtained from C57BL/6 mouse strain.

5. A hybrid cell vaccine according to any one of claims 1 to 4, wherein it elicits a cellular immune response which is a mixed Th1/Th2 cell response stimulating a Kinetoplastid Membrane Protein-11 specific CD8+ cytotoxic T cell response.

6. A hybrid cell vaccine according to any one of claims 1 to 5, wherein it stimulates an antibody response, a CTL response, T-cell proliferative response, IL-2 generation, reduction of splenic and liver parasite burden along with sterile cure against *Leishmania* upon therapeutic vaccination to a mammal.

7. A hybrid vaccine formulation against leishmanaisis comprising the hybrid cell vaccine according to claim 1 along with an adjuvant and/or cytokine.

8. A pharmaceutical formulation comprising a vaccine according to any one of the preceding claims along with a carrier or excipient.

9. A process for the preparation of the hybrid cell vaccine according to claim 1, against leishmaniasis comprising of the following steps:
[a] isolating macrophages from syngeneic BALB/c mice;
[b] transfecting the macrophages obtained in step [a] with Kinetoplastid Membrane Protein-11 cDNA containing mammalian expression vector pCM-LIC KMP-11;
[c] isolating dendritic cells from allogenic C57BL/6 mice;
[d] electro-fusing the transfected syngeneic macrophages obtained in step [b] with allogeneic dendritic cells obtained in step [c] to obtain fused hybrid cells to be used for therapeutic vaccination against leishmaniasis.

10. A process according to claim 9 wherein therapeutic administration of the hybrid cell vaccine is effective to eliminate parasites from organs of the mammals infected with *Leishmania.*

11. A process according to claim 9, wherein electrofusion is a fusion technique in which two different cell types: [a] syngeneic Bone Marrow Derived Macrophages expressing *Leishmania* antigen and [b] allogeneic dendritic cells are mixed at 1:1 ratio then aligned di-electrophoretically in a wax coated cuvette by applying 50V/cm alignment voltage which amounts to 20V for a cuvette of 0.4 cm path length by optimizing between 17 V and 25V along with simultaneous application of 625 V/cm square pulse voltage which amounts to 250 V for a cuvette of 0.4 cm path length by optimizing between 150V-300V for membrane fusion.

12. A hybrid vaccine formulation according to claim 7 for use in the treatment of leishmaniasis, wherein the administration of the vaccine is effective to eliminate the parasite from the organs of a mammal infected with *Leishmania.*

13. A hybrid vaccine formulation according to claim 12 for use in the treatment of leishmaniasis, wherein said formulation is inoculated intra-venously as a series of doses over a period of four weeks and the dose is 5 x 10⁵ cells/100g body wt./100 µl PBS with 5 days interval.

14. A hybrid vaccine formulation according to claim 12 or claim 13 for use in the treatment of leishmaniasis, wherein therapeutic administration of the vaccine is effective for obtaining complete clearance of splenic and hepatic parasite burden in late stage infection from the parasite.

## Patentansprüche

1. Hybridzellimpfstoff gegen Leishmaniose mit einem oder mehreren syngenen Makrophagen mit einem immunodominanten *Leishmania*-Antigen-Kinetoplastidmembranprotein-11, das mit einer oder mehreren allogenen dendritischen Zelle(n) elektrofusioniert ist.

2. Hybridzellimpfstoff nach Anspruch 1, wobei der Makrophage von BALB/c-Mäusen durch bekannte Verfahren erhalten wird.

3. Hybridzellimpfstoff nach Anspruch 1 oder Anspruch 2, wobei der *Leishmania-*Stamm *L*. *donovani* ist, aus dem das Gen von *Leishmania*-Antigen-Kinetoplastidmembranprotein-11 erhalten wird, und das Antigen ein immunodominantes Zelloberflächenprotein von *Leishmania*-Kinetoplastidmembranprotein-11 aufweist.

4. Hybridzellimpfstoff nach einem der Ansprüche 1 bis 3, wobei die allogene dendritische Zelle von Knochenmarkvorläufern oder aus Knochenmark stammenden dendritischen Zellen (BMDC), die vom C57BL/6-Mausstamm erhalten werden, erhalten wird.

5. Hybridzellimpfstoff nach einem der Ansprüche 1 bis 4, wobei er eine Zellenimmunreaktion hervorruft, die eine gemischte Th1/Th2-Zellen-Reaktion ist, die eine für das Kinetoplastidmembranprotein-11 spezifische Reaktion von zytotoxischen CD8+ T-Zellen stimuliert.

6. Hybridzellimpfstoff nach einem der Ansprüche 1 bis 5, wobei er eine Antikörperreaktion, eine CTL-Reaktion, eine T-Zellen-Vermehrungsreaktion, eine IL-2-Erzeugung, eine Verringerung einer Milz- und Leberparasitenbelastung zusammen mit einem sterilen Heilmittel gegen *Leishmania* bei therapeutischer Impfung in einen Säuger stimuliert.

7. Hybridimpfstoffformulierung gegen Leishmaniose mit dem Hybridzellimpfstoff nach Anspruch 1 zusammen mit einem Hilfsmittel und/oder Zytokin.

8. Pharmazeutische Formulierung mit einem Impfstoff nach einem der vorangehenden Ansprüche zusammen mit einem Träger oder Exzipienten.

9. Verfahren für die Zubereitung des Hybridzellimpfstoffs nach Anspruch 1 gegen Leishmaniose mit den folgenden Schritten:
[a] Isolieren von Makrophagen aus syngenen BALB/c-Mäusen;
[b] Transfizieren der in Schritt [a] erhaltenen Makrophagen mit cDNA von Kinetoplastidmembranprotein-11, die den Säugerexpressionsvektor pCM-LIC KMP-11 enthält;
[c] Isolieren von dendritischen Zellen von allogenen C57BL/6-Mäusen;
[d] Elektrofusionieren der in Schritt [b] erhaltenen transfizierten syngenen Makrophagen mit in Schritt [c] erhaltenen allogenen dendritischen Zellen, um fusionierte Hybridzellen zu erhalten, die für die therapeutische Impfung gegen Leishmaniose verwendet werden sollen.

10. Verfahren nach Anspruch 9, wobei die therapeutische Verabreichung des Hybridzellimpfstoffs wirksam ist, um Parasiten von Organen der mit *Leishmania* infizierten Säuger zu beseitigen.

11. Verfahren nach Anspruch 9, wobei die Elektrofusion eine Fusionstechnik ist, bei der zwei verschiedene Zelltypen: [a] syngene von Knochenmark stammende Makrophagen, die das *Leishmania*-Antigen exprimieren, und [b] allogene dendritische Zellen in einem 1:1-Verhältnis vermischt werden, dann dielektrophoretisch in einer mit Wachs beschichteten Küvette durch Anlegen einer Ausrichtungsspannung von 50 V/cm, die 20 V für eine Küvette mit einer Weglänge von 0,4 cm durch Optimieren zwischen 17 V und 25 V beträgt, zusammen mit gleichzeitigem Anlegen einer Rechteckimpulsspannung von 625 V/cm, die 250 V für eine Küvette mit einer Weglänge von 0,4 cm durch Optimieren zwischen 150 V und 300 V beträgt, für die Membranfusion ausgerichtet werden.

12. Hybridimpfstoffformulierung nach Anspruch 7 zur Verwendung bei der Behandlung von Leishmaniose, wobei die Verabreichung des Impfstoffs wirksam ist, um den Parasiten aus den Organen eines mit *Leishmania* infizierten Säugers zu beseitigen.

13. Hybridimpfstoffformulierung nach Anspruch 12 zur Verwendung bei der Behandlung von Leishmaniose, wobei die Formulierung intravenös als Reihe von Dosen über einen Zeitraum von vier Wochen geimpft wird und die Dosis 5 x 10⁵ Zellen/100 g Körpergewicht/100 µl PBS in einem Intervall von 5 Tagen ist.

14. Hybridimpfstoffformulierung nach Anspruch 12 oder Anspruch 13 zur Verwendung bei der Behandlung von Leishmaniose, wobei die therapeutische Verabreichung des Impfstoffs zum Erhalten einer vollständigen Beseitigung einer Milz- und Leberparasitenbelastung bei einer Infektion im Spätstadium vom Parasiten wirksam ist.

## Revendications

1. Vaccin à cellules hybrides contre la leishmaniose comprenant un ou plusieurs macrophages syngéniques avec un antigène KMP (Kinetoplastid Membrane Protein)-11 de *Leishmania* immuno-dominant rattaché par électrofusion à une ou plusieurs cellules dendritiques allogéniques.

2. Vaccin à cellules hybrides selon la revendication 1, dans lequel le macrophage est obtenu à partir de souris BALB/c par l'intermédiaire de procédés connus.

3. Vaccin à cellules hybrides selon la revendication 1 ou la revendication 2, dans lequel la souche de *Leishmania* est *L*. *donovani* à partir de laquelle un gène KMP-11 de Leishmania est obtenu et l'antigène comprend une protéine à surface cellulaire immuno-dominante de protéine KMP-11 de *Leishmania.*

4. Vaccin à cellules hybrides selon l'une quelconque des revendications 1 à 3, dans lequel la cellule dendritique allogénique est obtenue à partir de progénitures de moelle osseuse ou de cellules dendritiques dérivées de moelle osseuse (BMDC) obtenues à partir d'une souche de souris C57BL/6.

5. Vaccin à cellules hybride selon l'une quelconque des revendications 1 à 4, dans lequel on obtient une réponse immune cellulaire laquelle est une réponse cellulaire Th1/Th2 mélangée stimulant une réponse de cellules CD8+ cytotoxique T spécifique à une protéine KMP-11.

6. Vaccin à cellules hybrides selon l'une quelconque des revendications 1 à 5, dans lequel on stimule une réponse anticorps, une réponse CTL, une réponse proliférative de cellule T, une génération IL-2, une réduction de la charge splénique et hépatique du parasite conjointement avec une cure stérile contre la *Leishmania* lors d'une vaccination thérapeutique d'un mammifère.

7. Formulation de vaccin hybride contre la leishmaniose comprenant le vaccin à cellules hybrides selon la revendication 1 conjointement avec un adjuvant et/ou de la cytokine.

8. Formulation pharmaceutique comprenant un vaccin selon l'une quelconque des revendications précédentes conjointement avec un porteur ou un excipient.

9. Procédé pour la préparation du vaccin à cellules hybrides selon la revendication 1, contre la leishmaniose comprenant les étapes suivantes consistant à :
[a] isoler des macrophages de souris BALB/c syngéniques ;
[b] transfecter les macrophages obtenus à l'étape [a] avec un ADN-c KMP-11 contenant un vecteur d'expression mammifère pCM-LIC KMP-11 ;
[c] isoler des cellules dendritiques de souris C57BL/6 allogéniques ;
[d] rattacher par électrofusion les macrophages syngéniques transfectés obtenus à l'étape [b] à des cellules dendritiques allogéniques obtenues à l'étape [c] pour obtenir des cellules hybrides fusionnées à utiliser pour la vaccination thérapeutique contre la leishmaniose.

10. Procédé selon la revendication 9, dans lequel une administration thérapeutique du vaccin à cellules hybrides est efficace pour éliminer des parasites d'organes des mammifères infectés par la *leishmaniose.*

11. Procédé selon la revendication 9, dans lequel une électrofusion est une technique de fusion dans laquelle deux types de cellules différentes : [a] des macrophages dérivés de moelle osseuse syngénique exprimant un antigène *Leishmania* et [b] des cellules dendritiques allogéniques sont mélangées à un rapport de 1:1 puis alignées de manière diélectrophorétique dans une cuvette enduite de cire en appliquant une tension d'alignement de 50V/cm qui atteint 20V pour une cuvette d'une longueur de trajet de 0,4 cm en optimisant entre 17V et 25V conjointement avec une application simultanée d'une tension impulsionnelle carrée de 625V/cm qui atteint 250V pour une cuvette d'une longueur de trajet de 0,4 cm en optimisant entre 150V - 300V pour une fusion de membrane.

12. Formulation de vaccin hybride selon la revendication 7 à utiliser dans le traitement de la leishmaniose, dans laquelle l'administration du vaccin est efficace pour éliminer le parasite des organes d'un mammifère infecté par *Leishmania.*

13. Formulation de vaccin hybride selon la revendication 12 à utiliser dans le traitement de la leishmaniose, dans laquelle ladite formulation est inoculée en intraveineuse sous forme d'une série de doses sur une période de quatre semaines et la dose est de 5 x 10⁵ cellules/100g poids du corps/100 µl PBS avec un intervalle de 5 jours.

14. Formulation de vaccin hybride selon la revendication 12 ou la revendication 13 à utiliser dans le traitement de la leishmaniose, dans laquelle l'administration thérapeutique du vaccin est efficace pour obtenir une clairance complète de la charge splénique et hépatique du parasite dans le stade tardif de l'infection au parasite.
